# EUROPEAN PATENT APPLICATION

(11) **EP 4 309 587 A1**
(43) Date of publication of application: **24.01.2024**
(21) Application number: 22770852.6
(22) Date of filing: 21.01.2022
(51) Int. Cl.: A61B 8/14

(54) **ULTRASONIC DIAGNOSTIC DEVICE AND METHOD FOR CONTROLLING ULTRASONIC DIAGNOSTIC DEVICE**

(30) Priority: 17.03.2021 JP 2021043393
(71) Applicant: FUJIFILM Corporation, Tokyo 106-8620 (JP)
(72) Inventor: KOSHINO Riko, Tokyo 106-8620 (JP)
(74) Representative: Hughes, Andrea Michelle
(86) International application number: PCT/JP2022/002108
(87) International publication number: WO 2022/196090

(57) **Abstract**

An ultrasound diagnostic apparatus and a control method of an ultrasound diagnostic apparatus that enable a user to clearly check whether or not a breast of a subject is sufficiently scanned are provided. An ultrasound diagnostic apparatus 1 includes a sensor 4 that detects an inclined angle or a height position of the ultrasound probe 2, an image generation unit 13 that generates an ultrasound image based on a reception signal obtained by scanning a subject using, a scanning detection unit 16 that detects a start point and an end point of scanning of the breast of the subject with the ultrasound probe along one direction based on the inclined angle or the height position of the ultrasound probe 2, and a scanning completion determination unit 17 that determines whether scanning complying with a determined scanning pattern is completed or not completed by performing image analysis on an ultrasound image generated from a reception signal at the start point and on an ultrasound image generated from a reception signal at the end point.

## Description

### BACKGROUND OF THE INVENTION

### 1. Field of the Invention

The present invention relates to an ultrasound diagnostic apparatus and a control method of an ultrasound diagnostic apparatus used for examining a breast of a subject.

### 2. Description of the Related Art

In the related art, an ultrasound diagnostic apparatus that obtains a tomographic image inside a subject by scanning a body surface of the subject with an ultrasound probe has been known. A technology disclosed in JP2018-183448A that enables a user to securely examine the subject in examining the subject using the ultrasound diagnostic apparatus has been developed. JP2018-183448A discloses a technology for acquiring information related to a posture of the ultrasound probe via a sensor and for determining whether an imaged part of the subject is present on the left or the right of the subject based on the acquired information related to the posture of the ultrasound probe.

### SUMMARY OF THE INVENTION

In a case where a breast of the subject is examined using the ultrasound diagnostic apparatus, the user may, for example, overlook a scanning range and not be able to sufficiently scan the entire breast because of a size or the like of the breast of the subject. In the technology of JP2018-183448A, while whether the breast scanned with the ultrasound probe is a left or right breast of the subject can be determined, it is difficult to clearly check whether or not the user has sufficiently scanned the breast.

The present invention has been conceived to eliminate the problem of the related art, and an object thereof is to provide an ultrasound diagnostic apparatus and a control method of an ultrasound diagnostic apparatus with which whether or not a user has sufficiently scanned a breast of a subject can be clearly checked.

In order to achieve the object, an ultrasound diagnostic apparatus according to an aspect of the present invention comprises an ultrasound probe, a sensor that detects an inclined angle or a height position of the ultrasound probe, an image generation unit that generates an ultrasound image based on a reception signal obtained by scanning a breast of a subject using the ultrasound probe, a scanning detection unit that detects a start point and an end point of scanning of the breast of the subject with the ultrasound probe along one direction based on the inclined angle or the height position of the ultrasound probe detected by the sensor, and a scanning completion determination unit that determines whether scanning complying with a determined scanning pattern is completed or not completed by performing image analysis on an ultrasound image generated by the image generation unit based on a reception signal obtained at the start point detected by the scanning detection unit and on an ultrasound image generated by the image generation unit based on a reception signal obtained at the end point detected by the scanning detection unit.

The scanning completion determination unit may determine that scanning complying with the determined scanning pattern is completed in a case where an anatomical landmark positioned at one end of a scanning range complying with the determined scanning pattern is recognized from the ultrasound image generated by the image generation unit based on the reception signal obtained at the start point detected by the scanning detection unit and where an anatomical landmark positioned at the other end of the scanning range complying with the determined scanning pattern is recognized from the ultrasound image generated by the image generation unit based on the reception signal obtained at the end point detected by the scanning detection unit.

The ultrasound diagnostic apparatus may further comprise a notification unit that notifies a user in a case where the scanning completion determination unit determines that scanning complying with the determined scanning pattern is not completed.

In addition, the ultrasound diagnostic apparatus may further comprise a monitor, a graph generation unit that generates a graph representing a change in the inclined angle or the height position of the ultrasound probe detected by the sensor between the start point and the end point detected by the scanning detection unit and displays the graph on the monitor, and a display image selection unit that displays at least the ultrasound image generated by the image generation unit based on the reception signal obtained at the start point or the ultrasound image generated by the image generation unit based on the reception signal obtained at the end point on the monitor after the end point is detected by the scanning detection unit.

In a case where a designation point on the graph generated by the graph generation unit is designated by a user, the display image selection unit may display an ultrasound image generated by the image generation unit based on a reception signal obtained at an imaging position corresponding to the designation point, on the monitor.

In a case where a designation point on the graph generated by the graph generation unit is designated by a user, the display image selection unit may display ultrasound images having a plurality of frames generated by the image generation unit based on reception signals obtained at a plurality of imaging positions corresponding to a determined range on the graph including the designation point, on the monitor as a video.

In addition, the ultrasound diagnostic apparatus may further comprise a monitor, and a determination result display unit that displays the number of times of scanning with the ultrasound probe and a determination result of the scanning completion determination unit on the monitor.

In addition, the ultrasound diagnostic apparatus may further comprise a scanning combining unit that, in a case where scanning with the ultrasound probe is interrupted based on an instruction of a user and where scanning with the ultrasound probe is resumed based on an instruction of the user, combines the interrupted scanning and the resumed scanning into one scanning.

A control method of an ultrasound diagnostic apparatus according to another aspect of the present invention comprises detecting an inclined angle or a height position of an ultrasound probe via a sensor, generating an ultrasound image based on a reception signal obtained by scanning a subject using the ultrasound probe, detecting a start point and an end point of scanning of a breast of the subject with the ultrasound probe along one direction based on the inclined angle or the height position of the ultrasound probe detected by the sensor, and determining whether scanning complying with a determined scanning pattern is completed or not completed by performing image analysis on an ultrasound image generated based on a reception signal obtained at the detected start point and on an ultrasound image generated based on a reception signal obtained at the detected end point.

In the control method of the ultrasound diagnostic apparatus, a determination that scanning complying with the determined scanning pattern is completed may be made in a case where an anatomical landmark positioned at one end of a scanning range complying with the determined scanning pattern is recognized from the ultrasound image generated based on the reception signal obtained at the detected start point and where an anatomical landmark positioned at the other end of the scanning range complying with the determined scanning pattern is recognized from the ultrasound image generated based on the reception signal obtained at the detected end point.

In addition, in the control method of the ultrasound diagnostic apparatus, a notification may be issued to a user in a case where a determination that scanning complying with the determined scanning pattern is not completed is made.

In addition, in the control method of the ultrasound diagnostic apparatus, at least the ultrasound image generated based on the reception signal obtained at the start point or the ultrasound image generated based on the reception signal obtained at the end point may be selected from among ultrasound images having a plurality of frames generated based on reception signals obtained between the detected start point and the detected end point and be displayed on a monitor after the end point is detected.

In addition, in the control method of the ultrasound diagnostic apparatus, in a case where a designation point on a generated graph is designated by a user, an ultrasound image generated based on a reception signal obtained at an imaging position corresponding to the designation point may be displayed on the monitor.

In addition, in the control method of the ultrasound diagnostic apparatus, in a case where a designation point on a generated graph is designated by a user, ultrasound images having a plurality of frames generated based on reception signals obtained at a plurality of imaging positions corresponding to a determined range on the graph including the designation point may be displayed on the monitor as a video.

In addition, in the control method of the ultrasound diagnostic apparatus, the number of times of scanning with the ultrasound probe and a determination result as to whether scanning complying with the determined scanning pattern is completed or not completed may be displayed on a monitor.

In addition, in the control method of the ultrasound diagnostic apparatus, in a case where scanning with the ultrasound probe is interrupted based on an instruction of a user and where scanning with the ultrasound probe is resumed based on an instruction of the user, the interrupted scanning and the resumed scanning may be combined into one scanning.

According to the present invention, an ultrasound diagnostic apparatus comprises a sensor that detects an inclined angle or a height position of an ultrasound probe, an image generation unit that generates an ultrasound image based on a reception signal obtained by scanning a breast of a subject using the ultrasound probe, a scanning detection unit that detects a start point and an end point of scanning of the breast of the subject with the ultrasound probe along one direction based on the inclined angle or the height position of the ultrasound probe detected by the sensor, and a scanning completion determination unit that determines whether scanning complying with a determined scanning pattern is completed or not completed by performing image analysis on an ultrasound image generated by the image generation unit based on a reception signal obtained at the start point detected by the scanning detection unit and on an ultrasound image generated by the image generation unit based on a reception signal obtained at the end point detected by the scanning detection unit. Thus, a user can clearly check whether or not the breast of the subject is sufficiently scanned.

### BRIEF DESCRIPTION OF THE DRAWINGS

Fig. 1 is a block diagram illustrating a configuration of an ultrasound diagnostic apparatus according to Embodiment 1 of the present invention.
Fig. 2 is a block diagram illustrating a configuration of a transmission and reception circuit in Embodiment 1 of the present invention.
Fig. 3 is a block diagram illustrating a configuration of an image generation unit in Embodiment 1 of the present invention.
Fig. 4 is a diagram schematically illustrating an ultrasound probe moved on a breast of a subject in Embodiment 1 of the present invention.
Fig. 5 is a diagram illustrating an example of a notification message displayed on a monitor in Embodiment 1 of the present invention.
Fig. 6 is a flowchart illustrating operation of the ultrasound diagnostic apparatus according to Embodiment 1 of the present invention.
Fig. 7 is a block diagram illustrating a configuration of an ultrasound diagnostic apparatus according to Embodiment 2 of the present invention.
Fig. 8 is an example of a graph showing a change in time of a height position of the ultrasound probe detected in Embodiment 2 of the present invention.
Fig. 9 is another example of a graph showing a change in time of an inclined angle of the ultrasound probe detected in Embodiment 2 of the present invention.
Fig. 10 is a diagram illustrating a display example of a graph and an ultrasound image in Embodiment 2 of the present invention.
Fig. 11 is a diagram illustrating an example in which points on the graph are displayed in a highlighted manner in Embodiment 2 of the present invention.
Fig. 12 is a diagram illustrating another example in which points on the graph are displayed in a highlighted manner in Embodiment 3 of the present invention.
Fig. 13 is a diagram illustrating still another example in which points on the graph are displayed in a highlighted manner in Embodiment 2 of the present invention.
Fig. 14 is a block diagram illustrating a configuration of an ultrasound diagnostic apparatus according to Embodiment 3 of the present invention.
Fig. 15 is a diagram illustrating a display example of a determination result of scanning completion and the ultrasound image in Embodiment 3 of the present invention.
Fig. 16 is a block diagram illustrating a configuration of an ultrasound diagnostic apparatus according to Embodiment 4 of the present invention.

### DESCRIPTION OF THE PREFERRED EMBODIMENTS

Hereinafter, embodiments of the invention will be described with reference to the accompanying drawings.

The description of configuration requirements described below is provided based on the representative embodiment of the present invention, but the present invention is not limited to such an embodiment.

In the present specification, a numerical range represented using "to" means a range including the numerical values before and after "to" as a lower limit value and an upper limit value.

In the present specification, the terms "identical" and "same" include an error range generally allowed in the technical field.

### Embodiment 1

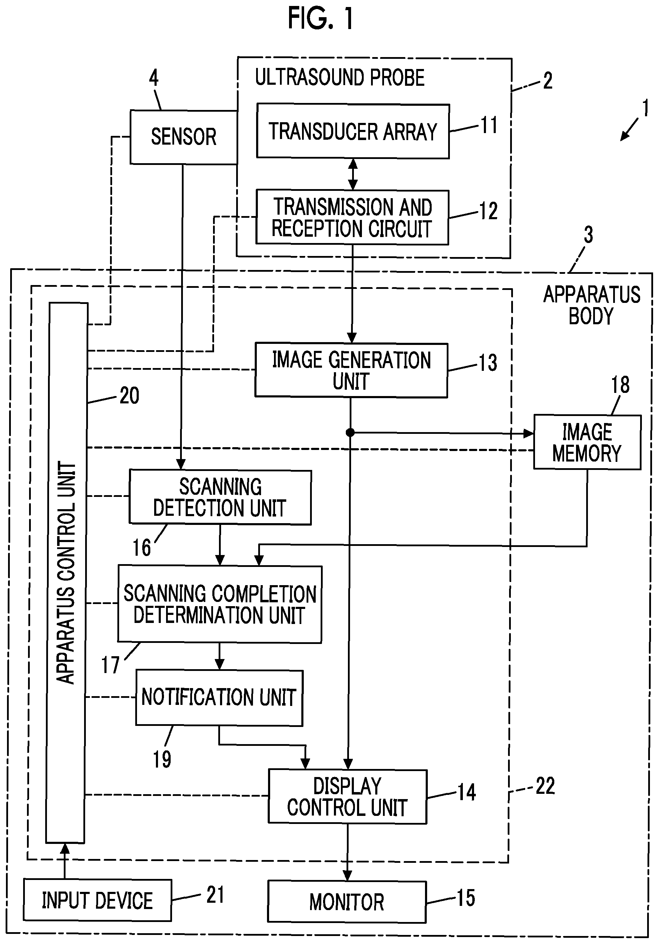

Fig. 1 illustrates a configuration of an ultrasound diagnostic apparatus 1 according to an embodiment of the present invention. The ultrasound diagnostic apparatus 1 comprises an ultrasound probe 2 and an apparatus body 3 connected to the ultrasound probe 2. In addition, a sensor 4 is attached to the ultrasound probe 2.

The ultrasound probe 2 comprises a transducer array 11. A transmission and reception circuit 12 is connected to the transducer array 11.

The apparatus body 3 comprises an image generation unit 13. The image generation unit 13 is connected to the transmission and reception circuit 12 of the ultrasound probe 2. In addition, a display control unit 14 and a monitor 15 are sequentially connected to the image generation unit 13. In addition, the apparatus body 3 comprises a scanning detection unit 16 connected to the sensor 4. In addition, a scanning completion determination unit 17 is connected to the scanning detection unit 16. In addition, a notification unit 19 is connected to the scanning completion determination unit 17, and the display control unit 14 is connected to the notification unit 19. In addition, an image memory 18 is connected to the image generation unit 13, and the scanning completion determination unit 17 is connected to the image memory 18.

In addition, an apparatus control unit 20 is connected to the sensor 4, the transmission and reception circuit 12, the image generation unit 13, the display control unit 14, the scanning detection unit 16, the scanning completion determination unit 17, the image memory 18, and the notification unit 19. In addition, an input device 21 is connected to the apparatus control unit 20.

In addition, a processor 22 for the ultrasound diagnostic apparatus 1 is composed of the image generation unit 13, the display control unit 14, the scanning detection unit 16, the scanning completion determination unit 17, the notification unit 19, and the apparatus control unit 20.

The transducer array 11 of the ultrasound probe 2 includes a plurality of one-dimensionally or two-dimensionally arranged ultrasound oscillators. Each of these ultrasound oscillators transmits an ultrasound wave in accordance with a drive signal supplied from the transmission and reception circuit 12 and receives an ultrasound echo from a subject to output a signal based on the ultrasound echo. Each ultrasound oscillator is configured by forming electrodes at both ends of a piezoelectric body consisting of, for example, a piezoelectric ceramic represented by lead zirconate titanate (PZT), a polymer piezoelectric element represented by polyvinylidene difluoride (PVDF), and a piezoelectric single crystal represented by lead magnesium niobate-lead titanate (PMN-PT).

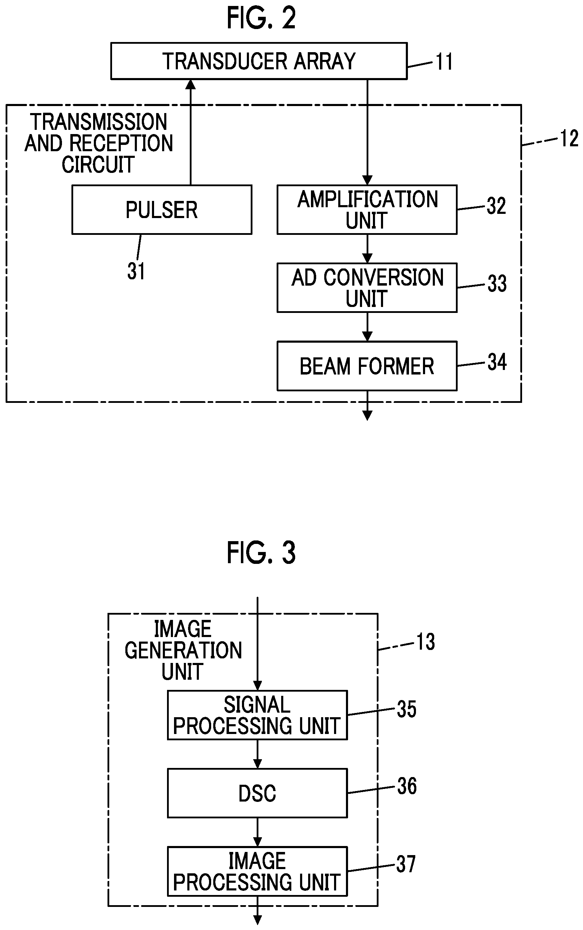

The transmission and reception circuit 12 transmits the ultrasound wave from the transducer array 11 and generates a sound ray signal based on a reception signal acquired by the transducer array 11 under control of the apparatus control unit 20. As illustrated in Fig. 2, the transmission and reception circuit 12 includes a pulser 31 connected to the transducer array 11 and an amplification unit 32, an analog digital (AD) conversion unit 33, and a beam former 34 sequentially connected in series from the transducer array 11.

The pulser 31 includes, for example, a plurality of pulse generators, adjusts a delay amount of each drive signal based on a transmission delay pattern selected in accordance with a control signal from the apparatus control unit 20 so that the ultrasound waves transmitted from the plurality of ultrasound oscillators of the transducer array 11 form an ultrasound beam, and supplies each drive signal to the plurality of ultrasound oscillators. In a case where a voltage having a pulse shape or a continuous wave shape is applied to the electrodes of the ultrasound oscillators of the transducer array 11, the piezoelectric bodies expand and contract to generate ultrasound waves having a pulse shape or a continuous wave shape from each ultrasound oscillator, and the ultrasound beam is formed from a combined wave of the ultrasound waves.

The transmitted ultrasound beam is reflected by, for example, a target such as a part of the subject and propagates toward the transducer array 11 of the ultrasound probe 2. The ultrasound echo propagating toward the transducer array 11 is received by each ultrasound oscillator constituting the transducer array 11. In this case, each ultrasound oscillator constituting the transducer array 11 receives the propagating ultrasound echo, expands and contracts to generate the reception signal that is an electric signal, and outputs the reception signal to the amplification unit 32.

The amplification unit 32 amplifies the signals input from each ultrasound oscillator constituting the transducer array 11 and transmits the amplified signals to the AD conversion unit 33. The AD conversion unit 33 converts the signals transmitted from the amplification unit 32 into digital reception data. The beam former 34 performs so-called reception focus processing of applying a delay to each reception data received from the AD conversion unit 33 and of adding each reception data together. By performing the reception focus processing, the sound ray signal in which each reception data converted by the AD conversion unit 33 is phased and added together and in which a focus of the ultrasound echo is narrowed is acquired.

The image generation unit 13 has a configuration in which a signal processing unit 35, a digital scan converter (DSC) 36, and an image processing unit 37 are sequentially connected in series as illustrated in Fig. 3.

The signal processing unit 35 corrects attenuation by distance of the sound ray signal received from the transmission and reception circuit 12 in accordance with depths of reflection positions of the ultrasound waves using a sound speed value set by the apparatus control unit 20 and then performs envelope detection processing on the sound ray signal to generate a B-mode image signal that is tomographic image information related to tissues inside the subj ect.

The DSC 36 converts the B-mode image signal generated by the signal processing unit 35 into an image signal complying with a scanning method of a typical television signal (raster conversion).

The image processing unit 37 performs various types of necessary image processing such as gradation processing on the B-mode image signal input from the DSC 36 and then transmits the B-mode image signal to the display control unit 14 and to the image memory 18. Hereinafter, the B-mode image signal on which the image processing is performed by the image processing unit 37 will be referred to as an ultrasound image.

The apparatus control unit 20 controls each part of the ultrasound probe 2, each part of the apparatus body 3, and the sensor 4 in accordance with a program and the like recorded in advance.

The display control unit 14 performs predetermined processing on the ultrasound image or the like generated by the image generation unit 13 and displays the ultrasound image or the like on the monitor 15 under the control of the apparatus control unit 20.

The monitor 15 performs various types of display under control of the display control unit 14. Examples of the monitor 15 include display devices such as a liquid crystal display (LCD) and an organic electroluminescence display (organic EL display).

The input device 21 is used for a user to perform an input operation. For example, the input device 21 is composed of a device used for the user to perform the input operation, such as a keyboard, a mouse, a trackball, a touchpad, and a touch panel.

The sensor 4 is used for detecting a height position or an inclined angle of the ultrasound probe 2. For example, an acceleration sensor or a gyro sensor may be used as the sensor 4.

Here, the height position of the ultrasound probe 2 is a height position of a tip part of the ultrasound probe 2 in contact with a body surface of the subject. For example, the sensor 4 can detect a value of a relative height position with reference to an arbitrary height in a case where the user moves the ultrasound probe 2. For example, the arbitrary height can be a height of a start point of scanning with the ultrasound probe 2.

In addition, the inclined angle of the ultrasound probe 2 is an inclined angle in a case where the ultrasound probe 2 is inclined in a plane orthogonal to a scan surface using the tip part of the ultrasound probe 2 as a fulcrum.

For example, the inclined angle in a state where the tip part of the ultrasound probe 2 is vertically downward can be set to zero. The sensor 4 can detect a positive value of the inclined angle in a case where inclination is performed to one side, and detect a negative value of the inclined angle in a case where inclination is performed to the other side.

While the ultrasound probe 2 is moved on the breast of the subject by the user along one direction in a front view of the subject, the scanning detection unit 16 detects a start point and an end point of scanning of the breast of the subject with the ultrasound probe 2 along one direction based on the height position or the inclined angle of the ultrasound probe 2 detected by the sensor 4.

Here, scanning of the breast of the subject with the ultrasound probe 2 along one direction means performing scanning in accordance with a determined scanning pattern on the breast of the subject while the user moves the ultrasound probe 2 along one direction in a front view of the subject in a state where the tip part of the ultrasound probe 2 is in contact with the body surface of the breast of the subject.

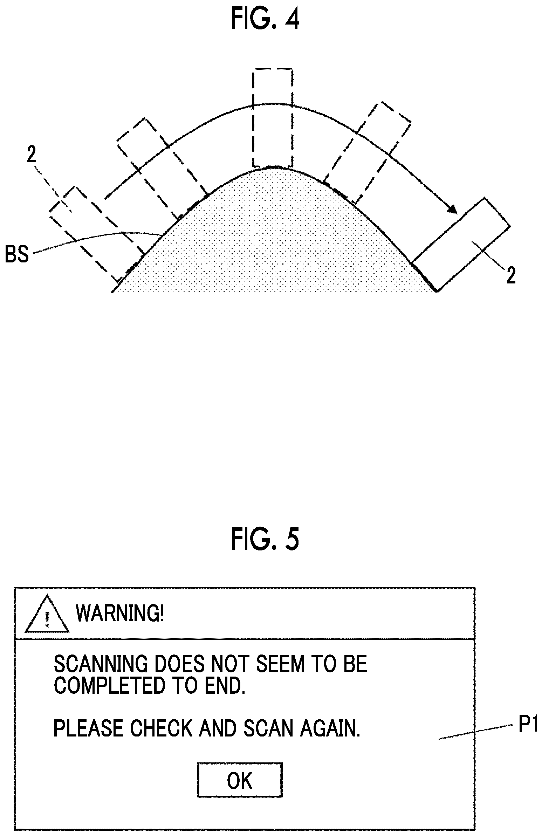

For example, the determined scanning pattern refers to a method of scanning the breast of the subject while moving the ultrasound probe 2 along one direction in a front view of the subject, such as a scanning method of scanning from one end part to the other end part of the breast of the subject along one direction in a front view of the subject or a scanning method of scanning from a nipple to a peripheral part of the breast of the subject along one direction in a front view of the subject. In Fig. 4, an example in which the ultrasound probe 2 moves on a body surface BS of the subject to scan a range from one end part to the other end part of the breast of the subject is illustrated as the determined scanning pattern.

Scanning from one end part to the other end part of the breast of the subject means scanning from one end part of the peripheral part of the breast of the subject to the other end part of the peripheral part of the breast while moving the ultrasound probe 2 along one direction. Here, moving the ultrasound probe 2 along one direction means that a movement direction of the ultrasound probe is constant. In addition, for example, the scanning method of scanning from one end part to the other end part of the breast of the subject includes a case of setting end parts of one breast in an up-down direction as one end part and the other end part or a case of setting end parts of one breast in a left-right direction as one end part and the other end part in a front view of the subject. In addition, for example, the scanning method of scanning from the nipple to the peripheral part includes a case of radially scanning from the nipple toward the peripheral part or a case of scanning by drawing a circle around the nipple in one direction using the nipple as a center.

Normally, the ultrasound probe 2 is generally moved at an almost constant speed during scanning of the breast of the subject. However, at the start of scanning, it is general to start moving from a standstill state or to start moving the ultrasound probe 2 that has been moving in a constant direction to a different direction. In addition, at the end of scanning, it is general to bring the ultrasound probe 2 to a standstill from a moving state or to start moving the ultrasound probe 2 that has been moving in a constant direction to a different direction. Accordingly, a value that significantly deviates from an originally detected value is generally obtained immediately before the start of scanning or immediately after the end of scanning.

Thus, for example, the scanning detection unit 16 can detect the start point of scanning with the ultrasound probe 2 based on a change in the detection value obtained by the sensor 4 after an elapse of a constant time from immediately before the start of scanning, such that the scanning detection unit 16 detects an imaging position immediately after a rapid change in the output value of the sensor 4 as the start point of scanning with the ultrasound probe 2. In addition, for example, the scanning detection unit 16 can detect the end point of scanning with the ultrasound probe 2 based on a change in the detection value obtained by the sensor 4 within a constant time from before the end of scanning to immediately after the end of scanning, such that the scanning detection unit 16 detects the imaging position immediately before a rapid change in the output value of the sensor 4 as the end point of scanning with the ultrasound probe 2.

The image memory 18 is a memory that stores at least an ultrasound image generated from the reception signals obtained in the ultrasound probe 2 at the start point of scanning detected by the scanning detection unit 16 or an ultrasound image generated from the reception signals obtained in the ultrasound probe 2 at the end point of scanning detected by the scanning detection unit 16 among ultrasound images sequentially generated by the image generation unit 13.

Hereinafter, for simplification of description, the ultrasound image generated by the image generation unit 13 based on the reception signals obtained at the start point of scanning will be referred to as the ultrasound image corresponding to the start point of scanning. The ultrasound image generated by the image generation unit 13 based on the reception signals obtained at the end point of scanning will be referred to as the ultrasound image corresponding to the end point of scanning.

For example, recording media such as a flash memory, a hard disk drive (HDD), a solid state drive (SSD), a flexible disk (FD), a magneto-optical disc (MO disc), a magnetic tape (MT), a random access memory (RAM), a compact disc (CD), a digital versatile disc (DVD), a secure digital card (SD card), and a universal serial bus memory (USB memory) can be used as the image memory 18.

The scanning completion determination unit 17 determines whether scanning of the breast of the subject complying with the determined scanning pattern is completed or not completed by reading out the ultrasound image corresponding to the start point of scanning detected by the scanning detection unit 16 and the ultrasound image corresponding to the end point of scanning detected by the scanning detection unit 16 from the image memory 18 and by performing image analysis on the ultrasound images.

Here, generally, it is known that an end part of a mammary gland is positioned near the peripheral part of the breast of the subject, a clavicle is positioned near the peripheral part of the breast positioned on a head side of the subject, an outer edge part of a latissimus dorsi is positioned near the peripheral part of the breast positioned on an armpit side of the subject, and a sternum is positioned near the peripheral part of the breast positioned on the opposite side to the armpit of the subject. That is, it is known that anatomical landmarks such as the end part of the mammary gland, the clavicle, the outer edge part of the latissimus dorsi, or the sternum are positioned near the peripheral part of the breast of the subject. In addition, anatomical landmarks such as a lactiferous duct are positioned near the nipple of the breast of the subject. Thus, various anatomical landmarks are positioned in and around the breast of the subject.

Thus, for example, in a case where an anatomical landmark positioned in one end part of a scanning range of the breast of the subject complying with the determined scanning pattern is recognized by performing image analysis on the ultrasound image corresponding to the start point of scanning detected by the scanning detection unit 16 and where an anatomical landmark positioned in the other end part of the scanning range of the breast of the subject complying with the determined scanning pattern is recognized by performing image analysis on the ultrasound image corresponding to the end point of scanning detected by the scanning detection unit 16, the scanning completion determination unit 17 can determine that scanning of the breast of the subject complying with the determined scanning pattern is completed.

In this case, in a case where an anatomical landmark positioned in one end part of a scanning range of the breast of the subject complying with the determined scanning pattern cannot be recognized from the ultrasound image corresponding to the start point of scanning detected by the scanning detection unit 16, or in a case where an anatomical landmark positioned in the other end part of the scanning range of the breast of the subject complying with the determined scanning pattern cannot be recognized from the ultrasound image corresponding to the end point of scanning detected by the scanning detection unit 16, the scanning completion determination unit 17 can determine that scanning of the breast of the subject complying with the determined scanning pattern is not completed.

In a case where the scanning completion determination unit 17 determines that scanning of the breast of the subject complying with the determined scanning pattern is not completed, the notification unit 19 notifies the user of the determination. For example, as illustrated in Fig. 5, the notification unit 19 can display, on the monitor 15, a notification panel P1 showing a message "Scanning does not seem to be completed to end. Please check and scan again." indicating that scanning with the ultrasound probe 2 is not completed. The user checks the message of the notification panel P1 and performs scanning complying with the determined scanning pattern again.

While the processor 22 including the image generation unit 13, the display control unit 14, the scanning detection unit 16, the scanning completion determination unit 17, the notification unit 19, and the apparatus control unit 20 is composed of a central processing unit (CPU) and of a control program causing the CPU to perform various types of processing, the processor 22 may be configured using a field programmable gate array (FPGA), a digital signal processor (DSP), an application specific integrated circuit (ASIC), a graphics processing unit (GPU), or other integrated circuits (IC) or may be composed of a combination thereof.

In addition, all or a part of the image generation unit 13, the display control unit 14, the scanning detection unit 16, the scanning completion determination unit 17, the notification unit 19, and the apparatus control unit 20 of the processor 22 can be configured to be integrated into one CPU or the like.

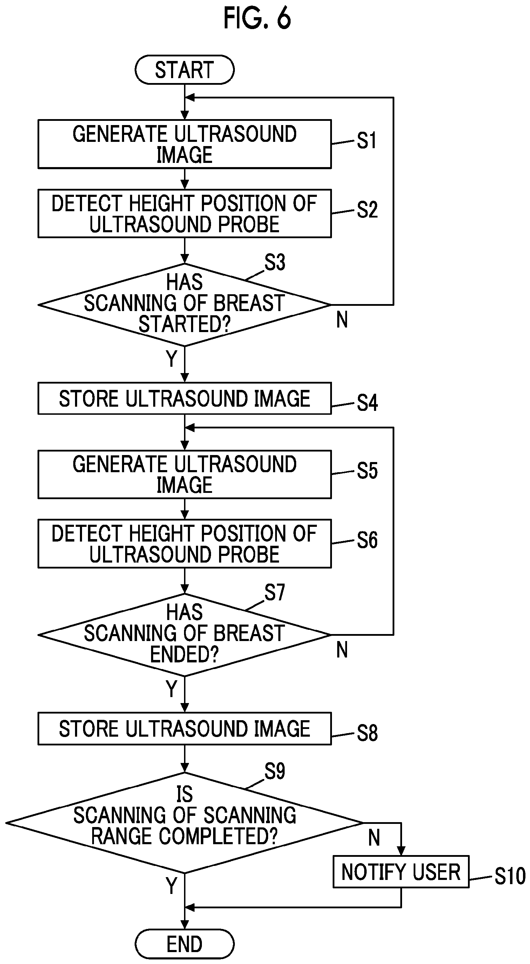

Next, operation of the ultrasound diagnostic apparatus 1 according to the embodiment of the present invention will be described using the flowchart illustrated in Fig. 6. Here, for example, the operation of the ultrasound diagnostic apparatus 1 in a case where the user scans the breast of the subject using the ultrasound probe 2 and where the height position of the ultrasound probe 2 is detected by the sensor 4 will be described.

First, the user positions the ultrasound probe 2 near one end part of the breast of the subject and starts moving the ultrasound probe 2 toward the other end part of the breast of the subject in one direction in a front view of the subject. In this state, an ultrasound image U1 is captured in step S1. In capturing the ultrasound image U1, the transmission and reception circuit 12 generates the sound ray signal by performing the so-called reception focus processing under control of the apparatus control unit 20. The sound ray signal generated by the transmission and reception circuit 12 is transmitted to the image generation unit 13. The image generation unit 13 generates the ultrasound image U1 using the sound ray signal transmitted from the transmission and reception circuit 12. The ultrasound image U1 generated in such a manner is transmitted to the display control unit 14 to be displayed on the monitor 15.

Next, in step S2, the height position of the ultrasound probe 2 is detected by the sensor 4.

Next, in step S3, the scanning detection unit 16 determines whether or not scanning has started by performing processing of detecting the start point of scanning of the breast of the subject along one direction based on a change in time of the height position of the ultrasound probe 2 detected in step S2.

In a case where scanning of the breast of the subject with the ultrasound probe 2 starts, normally, it is general to start moving the ultrasound probe 2 from a standstill state or to start moving the ultrasound probe 2 that has been moving in a constant direction to a different direction. Thus, for example, the scanning detection unit 16 can detect the start point of scanning with the ultrasound probe 2 based on a change in the detection value obtained by the sensor 4 after an elapse of a constant time from immediately before the start of scanning, such that the scanning detection unit 16 detects the imaging position immediately after a rapid change in the output value of the sensor 4 as the start point of scanning.

Here, the height position is detected only once in step S2. Thus, the scanning detection unit 16 cannot detect the start point of scanning in step S3 and determines that scanning has not started. In this case, a return is made to step S1 to newly generate the ultrasound image. Thus, processing of step S1 to step S3 is repeated until the scanning detection unit 16 detects the start of scanning of the breast of the subject after an elapse of a constant time required for detecting the start point of scanning via the scanning detection unit 16 in step S3.

In a case where the start of scanning is detected in step S3, a transition is made to step S4.

In step S4, the ultrasound image corresponding to the imaging position detected as the start point of scanning in step S3 is stored in the image memory 18.

Next, in step S5, an ultrasound image is generated in the same manner as step S1.

In step S6, the height position of the ultrasound probe 2 is detected in the same manner as step S2.

In step S7, the scanning detection unit 16 determines whether or not scanning has ended by performing processing of detecting the end point of scanning of the breast of the subject along one direction based on a change in time of the height position of the ultrasound probe 2 detected in step S6.

In a case where scanning of the breast of the subject with the ultrasound probe 2 ends, normally, it is general to bring the ultrasound probe 2 to a standstill from a moving state or to start moving the ultrasound probe 2 that has been moving in a constant direction to a different direction. Thus, the scanning detection unit 16 can detect the end point of scanning with the ultrasound probe 2 based on a change in the detection value obtained by the sensor 4 within a constant time from before the end of scanning to immediately after the end of scanning, such that the scanning detection unit 16 detects the imaging position immediately before a rapid change in the output value of the sensor 4 as the end point.

Here, the height position is detected only once in step S6. Thus, the scanning detection unit 16 cannot detect the end point of scanning in step S7 and determines that scanning has not ended. In this case, a return is made to step S5 to newly generate the ultrasound image. Thus, processing of step S5 to step S7 is repeated until the scanning detection unit 16 detects the end point of scanning of the breast of the subject after an elapse of a constant time required for detecting the end of scanning via the scanning detection unit 16 in step S7.

In a case where the end point of scanning of the breast of the subject is detected in step S7, a transition is made to step S8.

In step S8, the ultrasound image corresponding to the imaging position detected as the end point of scanning in step S7 is stored in the image memory 18.

Next, in step S9, the scanning completion determination unit 17 performs image analysis on the ultrasound image stored in the image memory 18 in step S4 and performs processing of recognizing an anatomical landmark positioned near the peripheral part of the breast from the ultrasound image. In addition, the scanning completion determination unit 17 performs image analysis on the ultrasound image stored in the image memory 18 in step S8 and performs processing of recognizing an anatomical landmark positioned near the peripheral part of the breast from the ultrasound image.

Furthermore, in step S9, the scanning completion determination unit 17 determines that scanning of the breast of the subject complying with the determined scanning pattern is completed in a case where an anatomical landmark is recognized from the image stored in step S4 and where an anatomical landmark is recognized from the image stored in step S8, and otherwise determines that scanning of the breast of the subject complying with the determined scanning pattern is not completed.

In a case where it is determined that scanning of the breast of the subject complying with the determined scanning pattern is completed in step S9, the operation of the ultrasound diagnostic apparatus 1 illustrated in the flowchart of Fig. 6 ends.

In addition, in a case where it is determined that scanning of the breast of the subject complying with the determined scanning pattern is not completed in step S9, a transition is made to step S10.

In step S10, the notification unit 19 notifies the user that scanning of the breast of the subject complying with the determined scanning pattern is not completed. For example, as illustrated in Fig. 5, the notification unit 19 can display, on the monitor 15, the notification panel P1 showing, for example, the message "Scanning does not seem to be completed to end. Please check and scan again." indicating that scanning with the ultrasound probe 2 is not completed.

In a case where processing of step S10 is completed in such a manner, the operation of the ultrasound diagnostic apparatus 1 illustrated in the flowchart of Fig. 6 ends. The user can check the message of the notification issued by the notification unit 19 in step S10 and scan the breast of the subject complying with the determined scanning pattern again.

As described above, according to the ultrasound diagnostic apparatus 1 according to Embodiment 1, the start point and the end point of scanning with the ultrasound probe 2 are detected by the scanning detection unit 16 based on the height position or the inclined angle of the ultrasound probe 2 detected by the sensor 4. In addition, whether or not scanning of the breast of the subject complying with the determined scanning pattern is completed is determined by performing image analysis on the ultrasound image corresponding to the start point of scanning and on the ultrasound image corresponding to the end point of scanning. Thus, the user can be prevented from overlooking the scanning range by clearly checking whether or not the breast of the subject as a target to be examined is sufficiently scanned and can sufficiently scan the breast of the subject.

While the height position of the ultrasound probe 2 is detected in step S2 after the ultrasound image is generated in step S1 in the flowchart illustrated in Fig. 6, processing of step S1 may be performed after processing of step S2 is performed, or processing of step S1 and processing of step S2 may be performed at the same time. In addition, while the height position of the ultrasound probe 2 is detected in step S6 after the ultrasound image is generated in step S5, processing of step S5 may be performed after processing of step S6 is performed, or processing of step S5 and processing of step S6 may be performed at the same time.

In addition, the ultrasound probe 2 and the apparatus body 3 can be connected via so-called wired communication or can be connected via so-called wireless communication.

In addition, while the sensor 4 is attached outside the ultrasound probe 2, the sensor 4 may be incorporated in the ultrasound probe 2.

In addition, the sensor 4 may not be attached to the ultrasound probe 2 as long as the height position or the inclined angle of the ultrasound probe 2 can be detected. For example, the sensor 4 can be attached to a hand of the subject holding the ultrasound probe 2. Even in this case, a relative value of the height position or a relative value of the inclined angle of the ultrasound probe 2 can be detected. Thus, the start point and the end point of scanning may be detected by the scanning detection unit 16 in the same manner as a case where the sensor 4 is directly attached to the ultrasound probe 2.

In addition, while the scanning pattern for scanning from one end part to the other end part of the breast of the subject has been illustrated, the scanning pattern applied to the present invention is not particularly limited thereto. For example, an arbitrary scanning pattern, such as a scanning pattern for scanning from the nipple to the peripheral part of the breast of the subject, determined by a facility such as a hospital in which examination is performed or by the user such as a doctor can be applied to the present invention.

In addition, the operation of the ultrasound diagnostic apparatus 1 according to the flowchart of Fig. 6 has ended in a case where the ultrasound image corresponding to the start point and the ultrasound image corresponding to the end point of scanning of the breast of the subject with the ultrasound probe 2 along one direction are stored and where it is determined that scanning complying with the determined scanning pattern is completed based on the stored ultrasound images. In a case where the user examines all of the breasts of the subject, a plurality of scanning ranges are scanned, and processing of step S1 to step S10 is performed at each scanning.

Accordingly, the user can detect all of the breasts of the subject without missing.

In addition, while the ultrasound images having two frames including the ultrasound image corresponding to the start point and the ultrasound image corresponding to the end point of scanning are stored in the image memory 18 in the operation of the ultrasound diagnostic apparatus 1 according to the flowchart of Fig. 6, ultrasound images having a plurality of frames obtained at timings before and after detection of the start point of scanning by the scanning detection unit 16 and ultrasound images having a plurality of frames obtained at timings before and after detection of the end point of scanning by the scanning detection unit 16 may be stored in the image memory 18.

In this case, the scanning completion determination unit 17 can determine whether or not the start point of scanning detected by the scanning detection unit 16 is an appropriate scanning start position complying with the determined scanning pattern by performing processing of recognizing an anatomical landmark positioned in one end part of the scanning range complying with the determined scanning pattern on the ultrasound images having the plurality of frames obtained at the timings before and after detection of the start point of scanning. In addition, the scanning completion determination unit 17 can determine whether or not the end point of scanning detected by the scanning detection unit 16 is an appropriate scanning end position complying with the determined scanning pattern by performing processing of recognizing an anatomical landmark positioned in the other end part of the scanning range complying with the determined scanning pattern on the ultrasound images having the plurality of frames obtained at the timings before and after detection of the end point of scanning. In such a manner, the scanning completion determination unit 17 can determine whether or not scanning complying with the determined scanning pattern is completed by performing image analysis on the ultrasound images having the plurality of frames.

### Embodiment 2

While the notification unit 19 notifies the user in a case where the scanning completion determination unit 17 determines that scanning of the breast of the subject complying with the determined scanning pattern is not completed in Embodiment 1, it is also possible to further display a graph representing a change in the height position or the inclined angle of the ultrasound probe 2 detected by the sensor 4 and the ultrasound image on the monitor 15 after scanning of the subject ends, that is, after the end point of scanning is detected by the scanning detection unit 16.

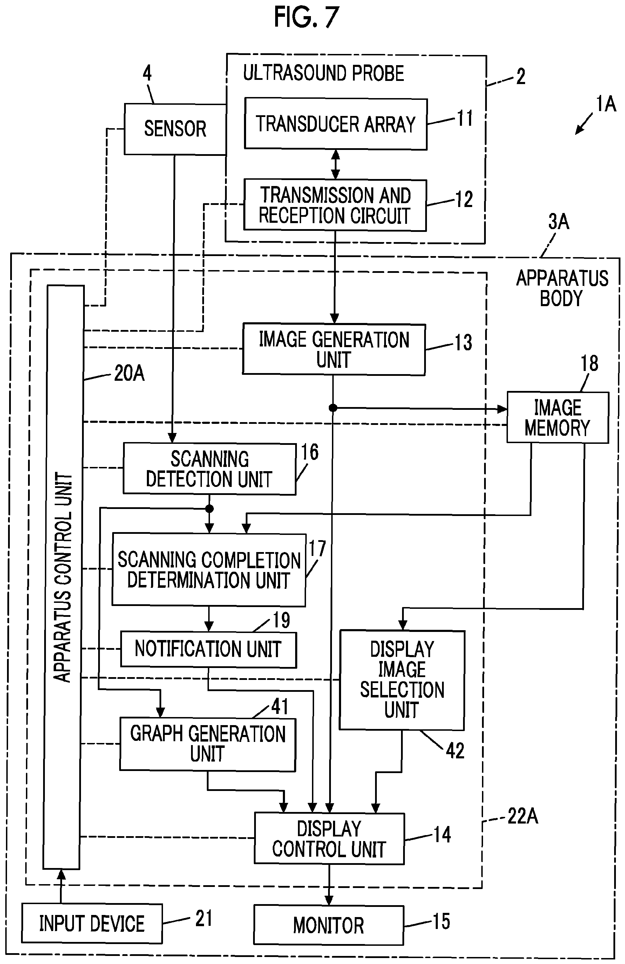

Fig. 7 illustrates a configuration of an ultrasound diagnostic apparatus 1A according to Embodiment 2. The ultrasound diagnostic apparatus 1A comprises an apparatus body 3A instead of the apparatus body 3 in the ultrasound diagnostic apparatus 1 of Embodiment 1 illustrated in Fig. 1. In addition, the apparatus body 3A includes a graph generation unit 41 and a display image selection unit 42 added to the apparatus body 3 in Embodiment 1, comprises an apparatus control unit 20A instead of the apparatus control unit 20, and comprises a processor 22A instead of the processor 22.

In the apparatus body 3A, the graph generation unit 41 is connected to the scanning detection unit 16, and the display control unit 14 and the apparatus control unit 20A are connected to the graph generation unit 41. In addition, the display image selection unit 42 is connected to the image memory 18, and the display control unit 14 and the apparatus control unit 20A are connected to the display image selection unit 42.

In addition, the processor 22A is composed of the image generation unit 13, the display control unit 14, the scanning detection unit 16, the scanning completion determination unit 17, the notification unit 19, the apparatus control unit 20A, the graph generation unit 41, and the display image selection unit 42.

The graph generation unit 41 generates a graph representing a change in time of the height position or the inclined angle of the ultrasound probe 2 detected by the sensor 4 between the start point and the end point of scanning with the ultrasound probe 2 detected by the scanning detection unit 16 and displays the generated graph on the monitor 15.

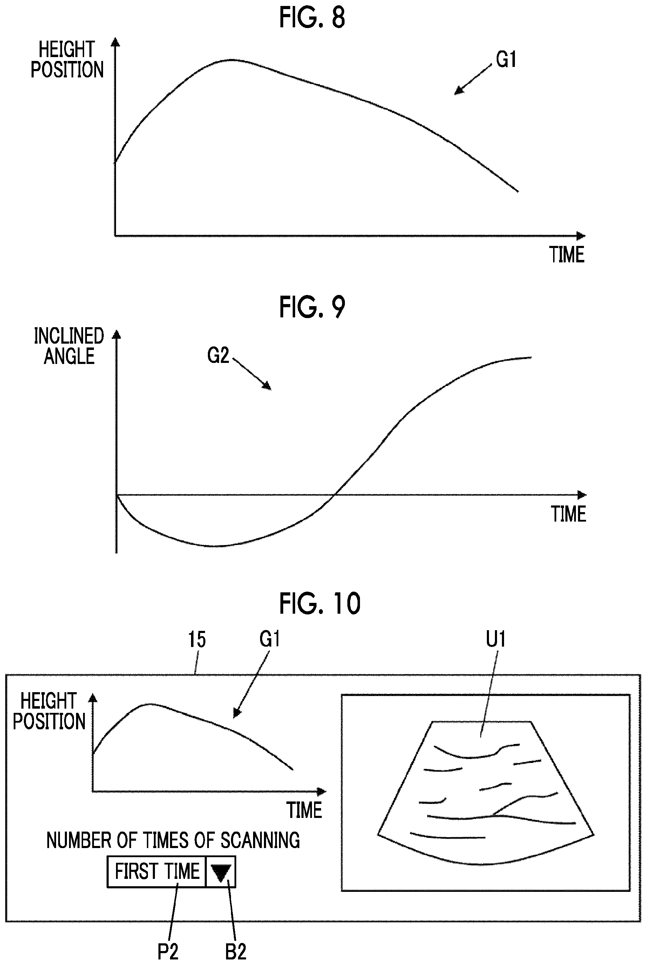

Fig. 8 illustrates an example of a graph G1 representing a change in time of the height position of the ultrasound probe 2 detected by the sensor 4 in a case of scanning from one end part to the other end part of the breast of the subject with the ultrasound probe 2. As time passes from a time point of zero, that is, a left end part of the graph G1 corresponding to the start point of scanning, the height position is gradually increased. The graph G1 has the maximum at a point corresponding to the nipple. Then, the height position is gradually decreased to a right end part of the graph G1 corresponding to the end point of scanning.

In addition, Fig. 9 illustrates an example of a graph G2 representing a change in time of the inclined angle of the ultrasound probe 2 detected by the sensor 4 in a case of scanning from one end part to the other end part of the breast of the subject with the ultrasound probe 2. In the graph G2, the inclined angle in a state where the tip part of the ultrasound probe 2 is vertically downward is set to zero. As time passes from a time point of zero, that is, a left end part of the graph G2 corresponding to the start point of scanning, the value of the inclined angle is increased in a negative direction and then is increased in a positive direction in the middle. The inclined angle reaches zero at a point corresponding to the nipple, and the value of the inclined angle is further increased in a positive direction.

In addition, for example, the graph generation unit 41 may display the graph G1 on the monitor 15 as illustrated in Fig. 10. In this case, the graph generation unit 41 may display, on the monitor 15, a scanning number-of-times display panel P2 showing the number of times that scanning is performed to generate the graph G1. The scanning number-of-times display panel P2 has a scanning number-of-times selection button B2. In a case where the scanning number-of-times selection button B2 is designated through the input device 21, a list of numbers of times that scanning has already been performed is displayed, and the user can select a desired number of times from the list. For example, in a case where the user selects "first time" from the list, the graph G1 generated in scanning performed for the first time is displayed on the monitor 15.

The display image selection unit 42 displays at least the ultrasound image generated by the image generation unit 13 based on the reception signals obtained at the start point of scanning or the ultrasound image generated by the image generation unit 13 based on the reception signals obtained at the end point of scanning on the monitor 15 after the end point of scanning is detected by the scanning detection unit 16.

Here, for example, as illustrated in Fig. 10, in a case where the graph G1 is displayed on the monitor 15 by the graph generation unit 41 and where the left end part of the graph G1 corresponding to the start point of scanning is designated by the user through the input device 21, the display image selection unit 42 may display the ultrasound image U1 corresponding to the start point of scanning on the monitor 15 from the image memory 18 based on input information provided by the user. In addition, while illustration is not provided, in a case where the right end part of the graph G1 corresponding to the end point of scanning is designated by the user through the input device 21, the display image selection unit 42 may display the ultrasound image corresponding to the end point of scanning on the monitor 15 from the image memory 18 based on the input information provided by the user.

As described above, according to the ultrasound diagnostic apparatus 1A according to Embodiment 2, the graph G1 representing a change in time of the height position or the inclined angle of the ultrasound probe 2 detected between the start point and the end point of scanning of the breast of the subject along one direction detected by the scanning detection unit 16 is displayed on the monitor 15. Furthermore, the ultrasound image U1 corresponding to the start point of scanning and the ultrasound image corresponding to the end point of scanning are displayed on the monitor 15. Thus, the user can be prevented from overlooking the scanning range by more clearly checking whether or not the breast of the subject as a target to be examined is sufficiently scanned and can sufficiently scan the breast of the subject.

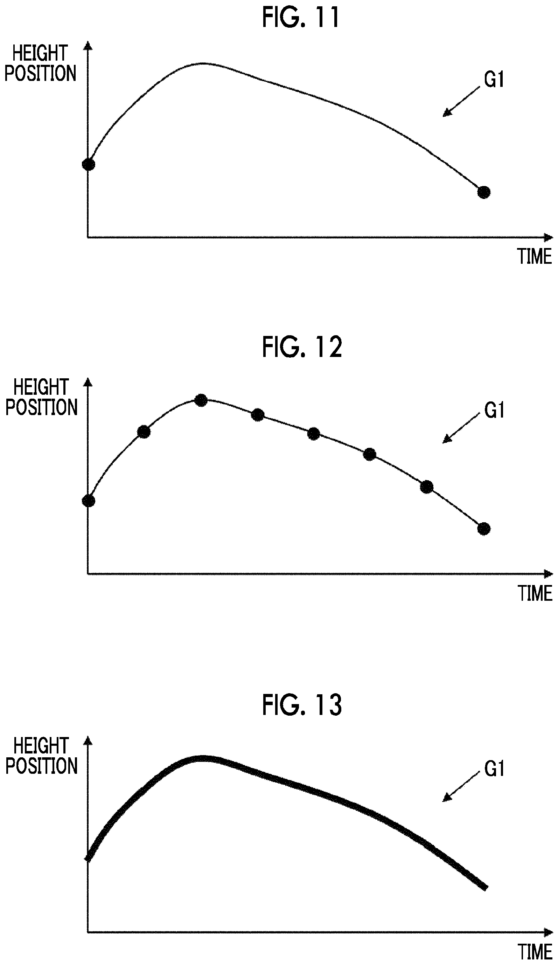

For example, as illustrated in Fig. 11, the graph generation unit 41 can display a point corresponding to the start point of scanning and a point corresponding to the end point of scanning of the breast of the subject in the graph G1 on the monitor 15 in a highlighted manner. Accordingly, the user can easily perceive the point on the graph G1 corresponding to the start point of scanning of the breast of the subject and the point on the graph G1 corresponding to the end point of scanning of the breast of the subject and easily designate the points.

In addition, while the ultrasound image U1 corresponding to the start point of scanning and the ultrasound image corresponding to the end point of scanning detected by the scanning detection unit 16 have been described as being stored in the image memory 18, an ultrasound image corresponding to an arbitrary imaging position between the start point and the end point of scanning detected by the scanning detection unit 16 may be further stored in the image memory 18.

For example, in addition to the ultrasound image U1 corresponding to the start point of scanning and the ultrasound image corresponding to the end point of scanning detected by the scanning detection unit 16, ultrasound images having a determined number of frames generated between the start point and the end point of scanning detected by the scanning detection unit 16 may be stored in the image memory 18.

In this case, for example, in a case where points corresponding to the ultrasound images having the determined number of frames on the graph G1 displayed on the monitor 15 are designated by the user through the input device 21, the display image selection unit 42 can display the corresponding ultrasound images on the monitor 15. Accordingly, the user can more clearly check whether or not the breast of the subject is sufficiently scanned.

In addition, in this case, for example, as illustrated in Fig. 12, the graph generation unit 41 can display the points corresponding to the ultrasound images having the determined number of frames stored in the image memory 18 on the graph G1 in a highlighted manner. Accordingly, the user can easily perceive which point is to be designated through the input device 21 in order to check the ultrasound image.

In addition, for example, all ultrasound images generated between the start point and the end point of scanning detected by the scanning detection unit 16 may be stored in the image memory 18. In this case, in a case where an arbitrary point on the graph G1 displayed on the monitor 15 is designated by the user through the input device 21, the display image selection unit 42 can display the corresponding ultrasound image on the monitor 15.

In addition, in this case, for example, as illustrated in Fig. 13, the graph generation unit 41 can display the points corresponding to the ultrasound images of all frames stored in the image memory 18 on the graph G1 in a highlighted manner.

In addition, in a case where ultrasound images having a plurality of frames generated between the start point and the end point of scanning are stored in the image memory 18 in addition to the ultrasound image U1 corresponding to the start point of scanning and to the ultrasound image corresponding to the end point of scanning, the ultrasound images having the plurality of frames may be continuously reproduced on the monitor 15 as a video instead of still images.

For example, in a case where the right end part of the graph G1 displayed on the monitor 15 is designated by the user through the input device 21, the ultrasound images having the plurality of frames are continuously displayed on the monitor 15 as a video in a reverse order from the ultrasound image corresponding to the end point of scanning to the ultrasound image corresponding to the start point of scanning. In addition, for example, in a case where the left end part of the graph G1 displayed on the monitor 15 is designated by the user through the input device 21, the ultrasound images having the plurality of frames are continuously displayed on the monitor 15 as a video from the ultrasound image corresponding to the start point of scanning to the ultrasound image corresponding to the end point of scanning.

In addition, for example, in a case where an arbitrary designation point present between a point corresponding to the start point of scanning and a point corresponding to the end point of scanning on the graph G1 is designated by the user through the input device 21, ultrasound images having a plurality of frames generated based on the reception signals obtained at a plurality of imaging positions corresponding to a determined range including the designation point on the graph G1 may be reproduced as a video.

The determined range corresponding to the ultrasound images having the plurality of frames on the graph G1 may be set by the user through the input device 21. For example, the determined range on the graph G1 may be set as an initial setting in a case where the ultrasound diagnostic apparatus 1A is used for the first time, and then, the initial setting may be maintained.

In addition, whether to reproduce the ultrasound images having the plurality of frames as a video in accordance with an elapse of time or to reproduce the ultrasound images having the plurality of frames in a reverse order of time may be set by the user through the input device 21. For example, this setting may also be set as an initial setting in a case where the ultrasound diagnostic apparatus 1A is used for the first time, and then, the initial setting may be maintained.

In addition, in a case where the user examines all of the breasts of the subject, a plurality of scanning ranges are scanned. In a case where scanning is performed a plurality of times in such a manner, a display of the scanning number-of-times display panel P2 illustrated in Fig. 10 is updated by the graph generation unit 17. For example, in a case where the number of times of scanning is one, the scanning number-of-times display panel P2 has only an item corresponding to scanning performed for the first time. In a case where the number of times of scanning reaches two, an item corresponding to scanning performed for the second time is added in addition to the item corresponding to scanning performed for the first time. Thus, the user can select an item corresponding to a desired number of times that scanning has already been performed from the scanning number-of-times display panel P2 and check a graph generated in scanning corresponding to the selected item on the monitor 15. Accordingly, the user can detect all of the breasts of the subject without missing.

### Embodiment 3

While displaying the graph G1 generated by the graph generation unit 41 on the monitor 15 has been described in Embodiment 2, a determination result of the scanning completion determination unit 17 may be displayed instead of the graph G1.

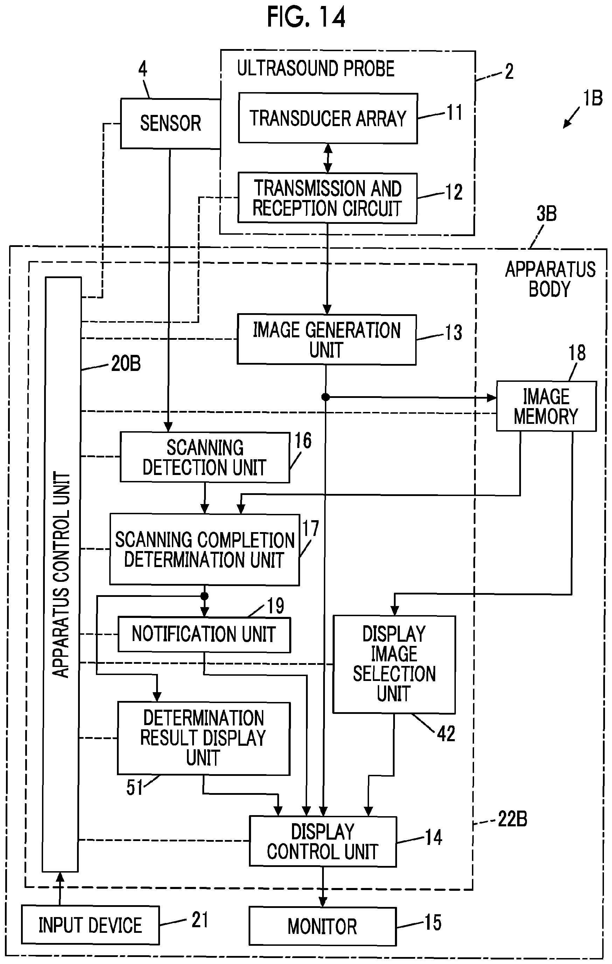

Fig. 14 illustrates a configuration of an ultrasound diagnostic apparatus 1B according to Embodiment 3. The ultrasound diagnostic apparatus 1B of Embodiment 3 comprises an apparatus body 3B instead of the apparatus body 3A in the ultrasound diagnostic apparatus 1A of Embodiment 2 illustrated in Fig. 7. The apparatus body 3B comprises a determination result display unit 51 instead of the graph generation unit 41, an apparatus control unit 20B instead of the apparatus control unit 20A, and a processor 22B instead of the processor 22A in the apparatus body 3A of Embodiment 2.

In the apparatus body 3B, the determination result display unit 51 is connected to the scanning completion determination unit 17, and the display control unit 14 and the apparatus control unit 20B are connected to the determination result display unit 51.

In addition, the processor 22B is composed of the image generation unit 13, the display control unit 14, the scanning detection unit 16, the scanning completion determination unit 17, the notification unit 19, the apparatus control unit 20B, the display image selection unit 42, and the determination result display unit 51.

The determination result display unit 51 displays the number of times of scanning with the ultrasound probe 2, that is, the number of times that scanning of the breast of the subject complying with the determined scanning pattern is performed, and the determination result of the scanning completion determination unit 17 on the monitor 15.

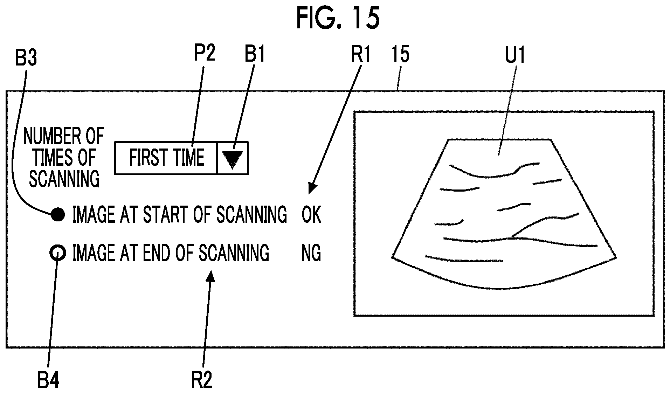

For example, as illustrated in Fig. 15, the determination result display unit 51 can display the scanning number-of-times display panel P2 and a message R1 representing whether or not an anatomical landmark is recognized in the ultrasound image corresponding to the start point of scanning and a message R2 representing whether or not an anatomical landmark is recognized in the ultrasound image corresponding to the end point of scanning on the monitor 15 as the determination result of the scanning completion determination unit 17. In the example in Fig. 15, "Image at start of scanning OK" representing that an anatomical landmark is recognized in the ultrasound image corresponding to the start point of scanning is displayed as the message R1, and "Image at end of scanning NG" representing that an anatomical landmark is not recognized in the ultrasound image corresponding to the end point of scanning is displayed as the message R2.

In addition, the determination result display unit 51 can display, near the message R1, a selection button B3 for displaying the ultrasound image U1 corresponding to the start point of scanning on the monitor 15 and display, near the message R2, a selection button B4 for displaying the ultrasound image corresponding to the end point of scanning on the monitor 15. In a case where the user selects the number of times of scanning of the determined scanning range of the breast of the subject from the scanning number-of-times display panel P2 and further selects one of the selection buttons B3 and B4 through the input device 21, the number of times of scanning selected by the user and the ultrasound image corresponding to the selection button B3 or B4 selected by the user are displayed on the monitor 15.

As described above, according to the ultrasound diagnostic apparatus 1B according to Embodiment 3, the determination result of the scanning completion determination unit 17 is displayed on the monitor 15 by the determination result display unit 51. Furthermore, the ultrasound image U1 corresponding to the start point of scanning and the ultrasound image corresponding to the end point of scanning are displayed on the monitor 15. Thus, the user can be prevented from overlooking the scanning range by more clearly checking whether or not the breast of the subject as a target to be examined is sufficiently scanned and can sufficiently scan the breast of the subject.

The messages R1 and R2 representing the determination result of the scanning completion determination unit 17 and the selection buttons B3 and B4 are displayed on the monitor 15 by the determination result display unit 51 in Embodiment 3. Instead, the selection button B3 for the user to display the ultrasound image U1 corresponding to the start point of scanning on the monitor 15 and a message "Image at start of scanning", and the selection button B4 for the user to display the ultrasound image corresponding to the end point of scanning on the monitor 15 and a message "Image at end of scanning" may be displayed on the monitor 15. Even in this case, the user can check the ultrasound image U1 corresponding to the start point of scanning and the ultrasound image corresponding to the end point of scanning. Thus, the user can be prevented from overlooking the scanning range by more clearly checking whether or not the breast of the subject as a target to be examined is sufficiently scanned and can sufficiently scan the breast of the subject.

### Embodiment 4

Scanning may be interrupted for some reason while the user is scanning the determined scanning range of the breast of the subject. In this case, scanning performed before and after the interruption may be combined into one to prevent erroneous detection of the end point of scanning of the breast of the subject complying with the determined scanning pattern.

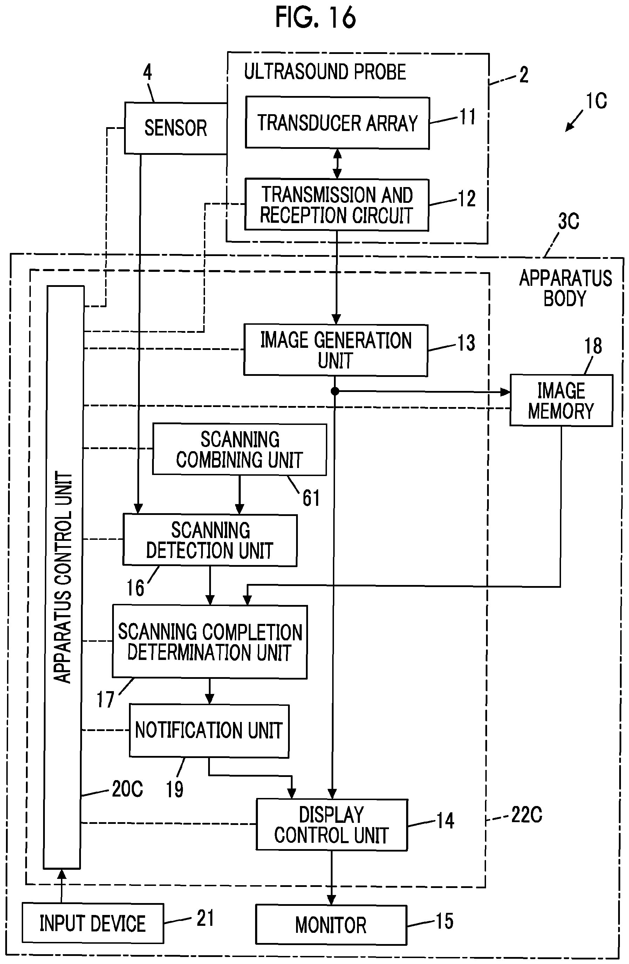

Fig. 16 illustrates a configuration of an ultrasound diagnostic apparatus 1C according to Embodiment 4. The ultrasound diagnostic apparatus 1C of Embodiment 4 comprises an apparatus body 3C instead of the apparatus body 3 in the ultrasound diagnostic apparatus 1 of Embodiment 1 illustrated in Fig. 1. In addition, the apparatus body 3C includes a scanning combining unit 61 added to the apparatus body 3 of Embodiment 1, comprises an apparatus control unit 20C instead of the apparatus control unit 20, and comprises a processor 22C instead of the processor 22.

In the apparatus body 3C, the scanning combining unit 61 is connected to the scanning detection unit 16 and to the apparatus control unit 20C.

In addition, the processor 22C is composed of the image generation unit 13, the display control unit 14, the scanning detection unit 16, the scanning completion determination unit 17, the notification unit 19, the apparatus control unit 20C, and the scanning combining unit 61.

In the ultrasound diagnostic apparatus 1C, scanning of the breast of the subject complying with the determined scanning pattern may be interrupted based on an instruction of the user provided through the input device 21. In addition, the interrupted scanning complying with the determined scanning pattern may be resumed based on an instruction of the user provided through the input device 21. For example, in a case where a scanning interruption button, not illustrated, is displayed on the monitor 15 and where the user selects the scanning interruption button through the input device 21, scanning complying with the determined scanning pattern is interrupted. In addition, for example, in a case where a scanning resumption button, not illustrated, is displayed on the monitor 15 and where the user selects the scanning resumption button through the input device 21, the interrupted scanning complying with the determined scanning pattern is resumed.

In a case where scanning of the breast of the subject complying with the determined scanning pattern is interrupted based on the instruction of the user provided through the input device 21 and where scanning complying with the determined scanning pattern is resumed based on the instruction of the user, the scanning combining unit 61 combines the interrupted scanning and the resumed scanning into one scanning.

For example, in a case where scanning of the breast of the subject complying with the determined scanning pattern is interrupted based on the instruction of the user, the scanning combining unit 61 causes the scanning detection unit 16 to temporarily stop the processing of detecting the end point of scanning complying with the determined scanning pattern. In addition, in a case where scanning of the breast of the subject complying with the determined scanning pattern is resumed based on the instruction of the user, the scanning combining unit 61 causes the scanning detection unit 16 to resume the processing of detecting the end point of scanning complying with the determined scanning pattern. Accordingly, the scanning detection unit 16 is prevented from erroneously detecting the end point of scanning complying with the determined scanning pattern in a case where scanning complying with the determined scanning pattern is interrupted.

As described above, according to the ultrasound diagnostic apparatus 1C of Embodiment 4, even in a case where scanning of the breast of the subject complying with the determined scanning pattern is interrupted, the interrupted scanning and the resumed scanning are combined into one scanning by the scanning combining unit 61, and the scanning detection unit 16 is prevented from erroneously detecting the end point of scanning of the breast of the subject complying with the determined scanning pattern. Thus, the user can be prevented from overlooking the scanning range by clearly checking whether or not the breast of the subject as a target to be examined is sufficiently scanned and can sufficiently scan the breast of the subject.

While the aspect of Embodiment 4 has been described as being applicable to Embodiment 1, the same application can be made to Embodiments 2 and 3.

### Explanation of References

1, 1A, 1B, 1C: ultrasound diagnostic apparatus
2: ultrasound probe
3, 3A, 3B, 3C: apparatus body
4: sensor
11: transducer array
12: transmission and reception circuit
13: image generation unit
14: display control unit
15: monitor
16: scanning detection unit
17: scanning completion determination unit
18: image memory
19: notification unit
20, 20A, 20B, 20C: apparatus control unit
21: input device
22, 22A, 22B, 22C: processor
31: pulser
32: amplification unit
33: AD conversion unit
34: beam former
35: signal processing unit
36: DSC
37: image processing unit
41: graph generation unit
42: display image selection unit
51: determination result display unit
61: scanning combining unit
B2: scanning number-of-times selection button
B3, B4: selection button
BS: body surface
G1, G2: graph
P1: notification panel
P2: scanning number-of-times display panel
R1, R2: message
U1: ultrasound image

## Claims

1. An ultrasound diagnostic apparatus comprising:
an ultrasound probe;
a sensor that detects an inclined angle or a height position of the ultrasound probe;
an image generation unit that generates an ultrasound image based on a reception signal obtained by scanning a breast of a subject using the ultrasound probe;
a scanning detection unit that detects a start point and an end point of scanning of the breast of the subject with the ultrasound probe along one direction based on the inclined angle or the height position of the ultrasound probe detected by the sensor; and
a scanning completion determination unit that determines whether scanning complying with a determined scanning pattern is completed or not completed by performing image analysis on an ultrasound image generated by the image generation unit based on a reception signal obtained at the start point detected by the scanning detection unit and on an ultrasound image generated by the image generation unit based on a reception signal obtained at the end point detected by the scanning detection unit.

2. The ultrasound diagnostic apparatus according to claim 1,
wherein the scanning completion determination unit determines that scanning complying with the determined scanning pattern is completed in a case where an anatomical landmark positioned at one end of a scanning range complying with the determined scanning pattern is recognized from the ultrasound image generated by the image generation unit based on the reception signal obtained at the start point detected by the scanning detection unit and where an anatomical landmark positioned at the other end of the scanning range complying with the determined scanning pattern is recognized from the ultrasound image generated by the image generation unit based on the reception signal obtained at the end point detected by the scanning detection unit.

3. The ultrasound diagnostic apparatus according to claim 1 or 2, further comprising:
a notification unit that notifies a user in a case where the scanning completion determination unit determines that scanning complying with the determined scanning pattern is not completed.

4. The ultrasound diagnostic apparatus according to any one of claims 1 to 3, further comprising:
a monitor;
a graph generation unit that generates a graph representing a change in the inclined angle or the height position of the ultrasound probe detected by the sensor between the start point and the end point detected by the scanning detection unit and displays the graph on the monitor; and
a display image selection unit that displays at least the ultrasound image generated by the image generation unit based on the reception signal obtained at the start point or the ultrasound image generated by the image generation unit based on the reception signal obtained at the end point on the monitor after the end point is detected by the scanning detection unit.

5. The ultrasound diagnostic apparatus according to claim 4,
wherein in a case where a designation point on the graph generated by the graph generation unit is designated by a user, the display image selection unit displays an ultrasound image generated by the image generation unit based on a reception signal obtained at an imaging position corresponding to the designation point, on the monitor.

6. The ultrasound diagnostic apparatus according to claim 4,
wherein in a case where a designation point on the graph generated by the graph generation unit is designated by a user, the display image selection unit displays ultrasound images having a plurality of frames generated by the image generation unit based on reception signals obtained at a plurality of imaging positions corresponding to a determined range on the graph including the designation point, on the monitor as a video.

7. The ultrasound diagnostic apparatus according to any one of claims 1 to 3, further comprising:
a monitor; and
a determination result display unit that displays the number of times of scanning with the ultrasound probe and a determination result of the scanning completion determination unit on the monitor.

8. The ultrasound diagnostic apparatus according to any one of claims 1 to 7, further comprising:
a scanning combining unit that, in a case where scanning with the ultrasound probe is interrupted based on an instruction of a user and where scanning with the ultrasound probe is resumed based on an instruction of the user, combines the interrupted scanning and the resumed scanning into one scanning.

9. A control method of an ultrasound diagnostic apparatus, the method comprising:
detecting an inclined angle or a height position of an ultrasound probe via a sensor;
generating an ultrasound image based on a reception signal obtained by scanning a subject using the ultrasound probe;
detecting a start point and an end point of scanning of a breast of the subject with the ultrasound probe along one direction based on the inclined angle or the height position of the ultrasound probe detected by the sensor; and
determining whether scanning complying with a determined scanning pattern is completed or not completed by performing image analysis on an ultrasound image generated based on a reception signal obtained at the detected start point and on an ultrasound image generated based on a reception signal obtained at the detected end point.

10. The control method of the ultrasound diagnostic apparatus according to claim 9,
wherein a determination that scanning complying with the determined scanning pattern is completed is made in a case where an anatomical landmark positioned at one end of a scanning range complying with the determined scanning pattern is recognized from the ultrasound image generated based on the reception signal obtained at the detected start point and where an anatomical landmark positioned at the other end of the scanning range complying with the determined scanning pattern is recognized from the ultrasound image generated based on the reception signal obtained at the detected end point.

11. The control method of the ultrasound diagnostic apparatus according to claim 9 or 10,
wherein a notification is issued to a user in a case where a determination that scanning complying with the determined scanning pattern is not completed is made.

12. The control method of the ultrasound diagnostic apparatus according to any one of claims 9 to 11,
wherein at least the ultrasound image generated based on the reception signal obtained at the start point or the ultrasound image generated based on the reception signal obtained at the end point is selected from among ultrasound images having a plurality of frames generated based on reception signals obtained between the detected start point and the detected end point and is displayed on a monitor after the end point is detected.

13. The control method of the ultrasound diagnostic apparatus according to claim 12,
wherein in a case where a designation point on a generated graph is designated by a user, an ultrasound image generated based on a reception signal obtained at an imaging position corresponding to the designation point is displayed on the monitor.

14. The control method of the ultrasound diagnostic apparatus according to claim 12,
wherein in a case where a designation point on a generated graph is designated by a user, ultrasound images having a plurality of frames generated based on reception signals obtained at a plurality of imaging positions corresponding to a determined range on the graph including the designation point are displayed on the monitor as a video.

15. The control method of the ultrasound diagnostic apparatus according to any one of claims 9 to 11,
wherein the number of times of scanning with the ultrasound probe and a determination result as to whether scanning complying with the determined scanning pattern is completed or not completed are displayed on a monitor.

16. The control method of the ultrasound diagnostic apparatus according to any one of claims 9 to 15,
wherein in a case where scanning with the ultrasound probe is interrupted based on an instruction of a user and where scanning with the ultrasound probe is resumed based on an instruction of the user, the interrupted scanning and the resumed scanning are combined into one scanning.
